(19) Europäisches Patentamt
European Patent Office
Office européen des brevets

(11) **EP 4 552 581 A1**

(12) **EUROPEAN PATENT APPLICATION**

(43) Date of publication:
**14.05.2025   Bulletin 2025/20**

(21) Application number: 23208577.9

(22) Date of filing: **08.11.2023**

(51) International Patent Classification (IPC):
**A61B 8/02** (2006.01)    **A61B 8/00** (2006.01)
**A61B 8/08** (2006.01)

(52) Cooperative Patent Classification (CPC):
**A61B 8/02; A61B 8/403; A61B 8/4227;**
**A61B 8/5223;** A61B 8/0883; A61B 8/0891

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **BOGATU, Laura Ioana**
  **5656AG Eindhoven (NL)**
• **SCHMITT, Lars**
  **5656AG Eindhoven (NL)**
• **MUEHLSTEFF, Jens**
  **5656AG Eindhoven (NL)**

(74) Representative: **Philips Intellectual Property &**
**Standards**
**High Tech Campus 52**
**5656 AG Eindhoven (NL)**

(54) **BLOOD PRESSURE MEASUREMENT METHOD AND SYSTEM**

(57)    A blood pressure measurement method and system comprising controlling an applied pressure cycle to a body part and simultaneously acquiring ultrasound data of an artery in the body part. An arterial area signal as a function of time during the pressure cycle is derived from the ultrasound data. The arterial area signal is fit to a pre-defined arterial collapse model which models arterial area as a function of transmural pressure. By using known values of applied pressure corresponding to each arterial area data point, a measure of arterial pressure is inferred by fitting the model, wherein the measure of arterial pressure is used as one of the model fitting parameters.

FIG. 6

EP 4 552 581 A1

**Description**

FIELD OF THE INVENTION

The present invention relates to the field of blood pressure measurement, in particular non-invasive blood pressure measurement.

BACKGROUND OF THE INVENTION

Blood pressure (BP) is a key physiological parameter for critical care and for cardiovascular disease management. BP is often measured using cuff-based oscillometry. This is referred to as non-invasive blood pressure (NIBP) measurement. However, this method can in some cases be inaccurate due to its indirect nature.

NIBP comprises inflating a cuff attached to a body part of a patient. The cuff applies a pressure. The applied pressure changes over the measurement cycle in accordance with an applied pressure cycle. The applied pressure alters the transmural pressure ($P_{tm}$) across the arterial wall. Blood pressure oscillations inside the artery cause arterial volume pulsations, amplitudes of which depend upon the $P_{tm}$. The arterial volume oscillations propagate through arm tissue, generating arm volume pulsations, which in turn generate pulsations inside the inflatable bladder of the attached cuff. These can be measured using a pressure transducer which transduces pressure changes inside the cuff. The pressure variations inside the cuff are referred to in shorthand as cuff pressure measurements (P_cuff).

The resulting cuff pressure oscillations are processed to derive systolic and diastolic BP values using algorithms which are well known.

The oscillometric principle therefore relies on indirect measurement of arterial oscillations because the arterial oscillations must first propagate through tissue, then through the cuff wall, before being sensed via pressure changes in the cuff. Sources of error and noise in this pressure transfer are therefore numerous.

SUMMARY OF THE INVENTION

Recent findings by the inventors have revealed several patient-specific characteristics which influence and interfere with the process by which arterial oscillations become expressed in cuff pressure measurements. Arm tissue is not homogeneous. Complex, subject-dependent muscle/fat distribution is present. Exact artery locations vary between subjects. Oscillations of superficial arteries are expressed differently in cuff pressure compared to oscillations of deeper arteries surrounded by subcutaneous fat.

Furthermore, the cuff itself has variable mechanical properties which affect pressure transfer. These vary from cuff to cuff and, for the same cuff, can vary depending upon how the cuff is applied. In general, the exact cuff transfer function is not known when measurement is performed.

Additional uncertainty also arises due to the length of the cuff. A cuff pressure pulsation is recorded as the pulse travels along the cuff length (e.g. ~14 cm), therefore the recorded cuff pressure waveform is a distorted version of the original arterial volume waveform. The resulting measured cuff pressure oscillation is particularly difficult to interpret also because transmural pressure is not uniform along the length of the cuff. It is unclear how to accurately characterize the artery area-to-$P_{tm}$ relationship by analysis of cuff pressure pulsations.

It has been the recognition of the inventors that the new evidence indicates that a direct and localized measurement of arterial pulsations may improve the accuracy and specificity of the BP measurement.

The basis of embodiments of the present invention is a further realization by the inventors that such direct measurement might be achieved via inference of one or more parameters from imaging modalities such as ultrasound.

Thus, embodiments of the present invention relate generally to ultrasound-based solutions and methods to overcome at least some of the above-described challenges associated with standard oscillometry.

The invention is defined by the claims.

According to examples in accordance with an aspect of the invention, there is provided a blood pressure measurement system, comprising: a pressure actuator for applying a varying pressure to a body part of a user; and an ultrasound sensing unit for acquiring ultrasound data of an artery in the body part during application of the pressure to the body part. There may further be provided a body mount unit adapted to releasably mount to the body of the user for holding the ultrasound sensing unit in a position for acquiring the ultrasound data. In this way ultrasound data can be acquired in a more stable fashion over the course of the measurement session.

There is further provided a processing device adapted to:

control the pressure actuator to execute a pre-defined applied pressure cycle, wherein an applied pressure to the body part is varied through a range of pressure levels;
during the applied pressure cycle, control the ultrasound sensing unit to acquire a time series of ultrasound data frames of the artery spanning the range of applied pressure levels;
apply a quantification algorithm to determine a measure indicative of a size of an arterial cross-sectional area in each frame; and
construct an artery area signal indicative of arterial cross-sectional area as a function of applied pressure using the extracted measures from each frame.

The processor may further be configured to retrieve a pre-determined arterial collapse model which relates arterial cross-sectional area, or a measure indicative thereof, with transmural pressure. In the model, transmural pressure may be expressed as a function of applied pressure by the pressure actuator and at least one measure of arterial pressure.

The processor may further be configured to fit parameters of the model to the artery area signal, wherein the parameters of the model include the at least one measure of arterial pressure.

The processor may further be configured to generate an output indicative of the at least one measure of arterial pressure based on the fitted parameters of the model.

The processing device by itself may also be provided as an aspect of the invention.

Optionally, the method may include a step of applying a segmentation algorithm to the ultrasound data to segment the artery cross-sectional area in the series of frames of the ultrasound data. The quantification algorithm might be applied following segmentation in some examples.

Thus, embodiments of the invention are based on measuring an arterial cross-sectional area over time over one or more heart cycles using ultrasound while simultaneously varying an applied pressure to the artery. Thus, the acquired artery area signal spans multiple different values of transmural pressure (due to the changing applied pressure). In particular, as the actuator pressure changes, a degree of collapse of the artery varies. When the cuff is at maximum pressure for example, the artery may be fully occluded or close to fully occluded. When the cuff is at minimum pressure, the artery may be only slightly occluded or not occluded at all. Thus, it will be recognized that a collapse state of the artery varies as a function of applied pressure. The ultrasound data can be used to track this over the measurement cycle, and an artery area signal obtained. Arterial collapse as a function of transmural pressure can be modelled a priori; models for this already exist in the literature. Arterial collapse in this context means change in artery cross-sectional area. Transmural pressure can be expressed in terms of a sum of external applied pressure and arterial pressure. Thus, by fitting observed arterial area changes as a function of applied pressure to the model, and using arterial pressure as one of the independent variables of the fitting operation, a measure of arterial pressure can be obtained. This measurement method offers improvements compared to the traditional approach due to the more direct observation of the artery via the ultrasound measurements and due to the use of the model. Inaccuracies and noise in the pressure signal are smoothed out via the fitting process.

With regards to the model fitting, this may comprise regression fitting, e.g. least squares regression fitting.

The measure of arterial pressure may be a measure which remains constant throughout measurement, e.g. systolic arterial pressure (SAP), mean arterial pressure (MAP), and/or diastolic blood pressure (DBP).

With regards to the optional body mount unit, in some embodiments, this may be, or may be comprised by, a cuff, wherein the pressure actuator and the ultrasound sensing unit are both integrated in the cuff. In other embodiments, the body mount unit could be a separate unit, for example a patch which integrates an ultrasound transducer unit comprised by the ultrasound sensing unit. This could be applied separately to the actuator. The actuator could be integrated in a cuff.

In some embodiments, the at least one measure of arterial pressure is systolic blood pressure or diastolic blood pressure.

In some embodiments, the arterial collapse model comprises a ratio of a logarithmic function of transmural pressure and a sigmoid function of transmural pressure.

In some embodiments, the arterial collapse model is represented by the expression:

$$A(P_{tm}) = (d \ln (a P_{tm} + B))/(1 + e^{\wedge}(- c P_{tm}))$$

where A represents blood vessel cross-sectional area, $P_{tm}$ represents transmural pressure, B is a constant, and wherein a, c, and d are tunable parameters of the model. When fitting the model, $P_{tm}$ may be expressed as a function of applied pressure, $P_{app}$, by the actuator and the at least one measure of arterial pressure, wherein the measure of arterial pressure is a further tunable parameter.

The parameter *a* may have units of 1/pressure, e.g. mmHg $^{-1}$. The parameter *c* may have units of 1/pressure, e.g. mmHg $^{-1}$. The parameter *d* may have units of distance, e.g. cm.

The constant B may be a dimensionless constant.

By way of example, the value of B may be set at 3.3.

This model is known in the literature for this field. For example, reference is made to the paper: Drzewiecki, G., Hood, R. & Apple, H. Theory of the oscillometric maximum and the systolic and diastolic detection ratios. Ann Biomed Eng 22, 88-96 (1994).

Referring to the above model expression, in some embodiments, when fitting the model, the arterial collapse model may be expressed as:

$$A = (d \ln (a (P_{art} - P_{app}) + B))/(1 + e^{-c (P_{art} - P_{app})})$$

where A represents blood vessel cross-sectional area, $P_{art}$ represents instantaneous arterial blood pressure, $P_{app}$ represents applied pressure by the actuator, and B is a constant.

Thus, here, transmural pressure, $P_{tm}$, has been expressed as the difference between arterial pressure and applied pressure, i.e. $P_{art}$ - $P_{app}$.

When fitting the model, $P_{art}$ may be expressed as $P_{art} = (P_{dia} + \Gamma \sin \omega t)$, where $\Gamma$ represents pulse pressure, $\omega$ represents heart rate, $t$ represents time of each arterial area measure, and wherein the measure of arterial pressure is $P_{dia}$, and wherein each of $P_{dia}$, $\Gamma$, $\omega$, a, c and d are tuneable parameters of the model. Since $t$ is a second independent variable, fitting the model could be done by multiple regression fitting. Alternatively, since $P_{app}$ is in practice a function of time, then, when fitting the model $P_{art}$ might be expressed instead as $P_{art} = (P_{dia} + \Gamma \sin \omega P_{app})$.

Instead of $P_{dia}$, $P_{sys}$ could instead be used.

In other words, $P_{art}$ is expressed as a sinusoidal function superposed on an offset defined by $P_{dia}$ or $P_{sys}$. This therefore takes account of the local oscillation of the arterial pressure over each heart cycle.

For fitting the models, the parameters $P_{dia}$, $\Gamma$, $\omega$, *a, c, d* are adjusted until the fitted arterial area model signal is as similar as possible to measured arterial area signal.

As an alternative, simply the upper or lower envelope of the measured arterial area signal could be fitted to the model. This avoids a need for the arterial collapse model to factor in the heart cycle oscillations in the area signal. In this case, the fitting the model may comprise extracting an upper envelope of the measured arterial area signal and fitting the upper envelope of the measured arterial area signal to the model of arterial collapse.

Here, for the fitting, the arterial collapse model may take the form:

$$A_{env-up} = d \ ln(a (P_{sys} - P_{app}) + B)/ (1 + exp(-c(P_{sys} - P_{app})))$$

where $A_{env-up}$ represents the upper envelope of the blood vessel cross-sectional area signal, $P_{sys}$ represents systolic blood pressure, $P_{app}$ represents applied pressure by the actuator, and B is a constant. The measure of arterial pressure to be derived from the model in this case is systolic blood pressure, $P_{sys}$. Each of $P_{sys}$, a, c and d are tuneable parameters of the model.

Instead of the upper envelope, the lower envelope could be used. Here, the fitting of the model may comprise extracting a lower envelope of the measured arterial area signal, and fitting the lower envelope of the measured arterial area signal to the model of arterial collapse. For the fitting, the arterial collapse model may take the form:

$$A_{env-low} = d \ ln(a (P_{dia} - P_{app}) + B)/ (1 + exp(-c(P_{dia} - P_{app})))$$

where $A_{env-low}$ represents the lower envelope of the blood vessel cross-sectional area signal, $P_{dia}$ represents diastolic blood pressure, and $P_{app}$ represents applied pressure by the actuator, and B is a constant. The measure of arterial pressure to be derived from the model in this case is diastolic blood pressure, $P_{dia}$. Each of $P_{dia}$, a, c and d are tuneable parameters of the model.

In some embodiments, fitting the model may comprise a first fitting operation and a second fitting operation, wherein the first fitting operation comprises fitting the upper envelope of the measured arterial area signal to the model of arterial collapse and the second fitting operation comprises fitting the lower envelope of the measured arterial area signal to the model of arterial collapse.

With regards to the ultrasound sensor unit, this may comprise an ultrasound transducer array. This allows for beamforming and/or beam-steering for example.

In some embodiments, the processing device may be adapted, during the applied pressure cycle, to acquire a respective time series of ultrasound data frames for each of a plurality of longitudinal locations along the artery.

In other words, the processing device may be adapted, during the applied pressure cycle, to acquire a respective time series of ultrasound data frames for each of a plurality of spatial planes through the artery, wherein the plurality of spatial planes are spaced from one another along a direction of a longitudinal length of the artery. Each may define a cross-section through the blood vessel carried by the artery. Each intersects the longitudinal axis of the blood vessel.

In some embodiments, the processing device may be adapted to construct an artery area signal as a function of time for each of the longitudinal location, i.e. for each of the spatial planes. In some embodiments, the arterial collapse model may be fitted to the respective artery area signal for each of the spatial planes. Alternatively, an average area signal could be derived from the plurality of artery area signals and the arterial collapse model fitted to this average area signal.

The acquisition of an arterial area signal for multiple locations (multiple planes) along the length of artery beneath the cuff can in general be useful for adapting calculations in accordance with variation in pressure transfer between the cuff and the artery along the cuff length. Different sections of the artery may be subject to slightly different applied pressures during a single actuation state of the actuator (e.g. a single inflation state of a cuff comprising the actuator). Taking multiple measurements allows for this variation to be accounted for, either by factoring it in explicitly, or by averaging.

In some embodiments, an operation is proposed wherein the multiple area signals are used to infer a corrected function of transmural pressure vs time at each plane location based on an assumption that measurements at a most central location are the most accurate.

In particular, in some embodiments, the processing device may be adapted to construct an artery area signal as a function of time for each of the aforementioned spatial planes. The system may comprise a cuff for attachment to an arm (or other body part) of the patient, wherein the cuff comprises the pressure actuator, and wherein the cuff, when worn, has a length for extending along a longitudinal direction of the artery, and wherein the applied pressure is applied along at least a section of the length of the cuff. The processing device may be adapted to fit the arterial collapse model to a first of the plurality of artery area signals, for example wherein the first corresponds to a central location along the length of the cuff, to derive a first fitted model. The processing device may be further adapted to use the first fitted model to infer a transmural pressure vs time signal for the locations of each of the other of the plurality of spatial planes, based on the respective artery area signal for each spatial plane, and based on an assumption that the same fitted model applies to each location, to thereby derive a respective transmural pressure vs time signal for each of the locations of the plurality of spatial planes.

Here, the assumption is that the transmural pressure is distorted at edge regions of the cuff due to physical inhomogeneity of pressure transfer to tissue from different regions of the cuff. This is based on empirical findings of the inventors, as will be detailed later.

It is therefore proposed to fit the model for a central location. The findings of the inventors show that the central location has the least distortion in the pressure transfer between cuff and blood vessel. From this, transmural pressure as a function of time can be inferred at the other locations by assuming that the central fitted model is correct and that the area measurements at the other locations are correct.

In order to derive at least one measure of arterial pressure, in some embodiments, a method may be employed which utilizes pulse arrival time (PAT) measurements taken at a peripheral arterial site, preferably arterially downstream from the artery which is lying beneath the cuff and whose area is measured by the ultrasound data. This method is detailed in the document EP4173556A1.

This method employs a pre-determined model of a measure of arterial pressure as a function of measured PAT, such that by measuring PAT, the measure of arterial pressure can be inferred. The transfer function between the measure of arterial pressure and PAT has a dependency upon a calibration factor. Acquisition of the calibration factor involves measuring a transmural pressure for an artery as a function of time for each of a series of different applied pressures to the artery. It is the realization of the inventors that the new concepts employed in accordance with the present invention may be advantageously applied to improve the PAT-based blood pressure inference method.

Thus, it is proposed that in some embodiments, the processing device may be further adapted to retrieve a model of a measure of arterial pressure as a function of pulse arrival time (PAT), wherein the model relates PAT to the measure of arterial pressure as a function of a calibration factor. The processing device may be further adapted to compute a value of the calibration factor based on the aforementioned plurality of transmural pressure vs time signals for the locations of the plurality of spatial planes.

The processing device may be further adapted to: receive a real-time PPG signal (e.g. corresponding to a location on the body, e.g. corresponding to a peripheral location on the body, e.g. the hand or finger) and a real-time ECG signal; compute a real-time PAT signal based on the PPG signal and ECG signal; and use the model of the measure of arterial pressure as a function of PAT to convert the PAT signal to a real-time blood pressure signal.

In some embodiments, the cuff may be adapted to improve homogeneity of pressure application across the length of the artery section which lies beneath the cuff. For example, in some embodiments, the system comprises a cuff for attachment to an arm of the patient, wherein the cuff comprises the pressure actuator, wherein the cuff, when worn, has a length for extending along a longitudinal direction of the artery, and wherein the applied pressure is applied along at least a section of the length of the cuff. In some embodiments, the actuator may be provided comprising a plurality of actuator elements, each element arranged for applying pressure to the artery across a different respective section of the length of the cuff, and each having an independently controllable applied pressure. This way, applied pressure can potentially be controlled differently at different of the respective length sections.

In some embodiments, the plurality of spatial planes referred to in the description of previous embodiments may include at least one spatial plane aligned longitudinally within each of the aforementioned length sections of the cuff. In other words, a respective arterial area signal may be acquired at a respective longitudinal location corresponding to a longitudinal location of each of the actuator elements.

In some embodiments, the processing device may be adapted to control the applied pressures of the plurality of actuator elements such that an artery area measured within each length section at any given time during the applied pressure cycle is the same.

For example, the actuator may comprise an inflatable bladder, and wherein the inflatable bladder comprises a plurality of bladder sub-chambers (or 'cuff compartments'), each sub-chamber arranged for applying pressure to the artery across a different respective section of the length of the cuff, and each having an independently controllable inflation level.

Another finding of the inventors is that lateral displacement of tissue differs at different points along the length of the cuff, and it is preferred to acquire measurements using data acquired at a location of least lateral displacement. Thus, in some embodiments, the processing device may be adapted to: analyze the time series of ultrasound data frames for each of the plurality of spatial planes through the artery to compute a measure of longitudinal tissue displacement at a longitudinal location of each of the spatial planes; identify the one of the spatial planes for which longitudinal tissue motion is minimum; and perform the fitting of the arterial collapse model and the computation of the at least one measure of arterial pressure using the ultrasound data from the location of said identified one of the spatial planes.

Another aspect of the invention is a blood pressure measurement method, the method comprising communicating with: a pressure actuator for applying a varying pressure to a body part of a user, and an ultrasound sensing unit for acquiring ultrasound data of an artery in the body part during application of the pressure to the body part.

The method comprises controlling the pressure actuator to execute a pre-defined applied pressure cycle, wherein an applied pressure to the body part is varied through a range of pressure levels.

The method further comprises, during the applied pressure cycle, controlling the ultrasound sensing unit to acquire a time series of ultrasound data frames of the artery spanning the range of applied pressure levels.

The method may optionally further comprise applying a segmentation algorithm to the ultrasound data to segment an artery cross-sectional area in the series of frames of the ultrasound data.

The method further comprises applying a quantification algorithm to determine a measure indicative of a size of an arterial cross-sectional area in each frame.

The method further comprises constructing an artery area signal indicative of arterial cross-sectional area as a function of applied pressure using the extracted measures from each frame.

The method further comprises retrieving a pre-determined arterial collapse model which relates arterial cross-sectional area, or a measure indicative thereof, with transmural pressure, wherein transmural pressure is expressed as a function of applied pressure by the pressure actuator and at least one measure of arterial pressure.

The method further comprises fitting parameters of the model to the artery area signal, wherein the parameters of the model include the at least one measure of arterial pressure.

The method further comprises generating an output indicative of the at least one measure of arterial pressure based on the fitted parameters of the model.

The method may be computer implemented. The method may be machine-implemented. The method is not a mental method.

Another aspect of the invention is a computer program product comprising computer program code configured to cause execution of a method in accordance with any embodiment described in this disclosure or in accordance with any claim of this application when run on a processor which is operatively coupled with: a pressure actuator for applying a varying pressure to a body part of a user; and an ultrasound sensing unit for acquiring ultrasound data of an artery in the body part during application of the pressure to the body part.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates sample MRI images obtained during a study which illustrate non-homogeneous compression of arm tissue by an NIBP cuff;
Fig. 2 illustrates sample MRI images obtained during a study which illustrate differing arterial position and branching arterial structure in different subjects;
Fig. 3 illustrates sample MRI images obtained during a study which illustrate differing rates of arterial collapse as a function of applied pressure at a central location of an NIBP cuff compared with a distal location of the cuff;
Fig. 4 illustrates arterial cross-sectional area of a brachial artery as a function of location along the length of an NIBP cuff when the arm is under compression by the NIBP cuff;
Fig. 5 outlines steps of an example method in accordance with one or more embodiments of the invention;
Fig. 6 is a block diagram of an example processing device and system in accordance with one or more embodiments of the invention;

Fig. 7 and Fig. 8 illustrate a comparison of arterial oscillation signals that can be obtained using standard cuff pressure measurements as compared with using ultrasound-based measurements;

Fig. 9 illustrates a model fit of an arterial collapse model in accordance with one or more embodiments of the invention; and

Fig. 10 illustrates an example cuff comprising a pressure actuator and comprising a plurality of ultrasound transducer units disposed as a series of longitudinal positions along the length of the cuff.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a blood pressure measurement method comprising controlling an applied pressure cycle to a body part and simultaneously acquiring ultrasound data of an artery in the body part. An arterial area signal as a function of time during the pressure cycle is derived from the ultrasound data. The arterial area signal is fit to a pre-defined arterial collapse model which models arterial area as a function of transmural pressure. In some cases, the arterial area may be derived/estimated from an arterial diameter measurement, or an arterial diameter measurement may be used as a proxy for the area signal. By using known values of applied pressure corresponding to each arterial area data point, a measure of arterial pressure can be inferred by fitting the model, wherein the measure of arterial pressure is used as one of the fitting parameters.

The making of the present invention was inspired by new research performed by the inventors into the mechanical interaction between a non-invasive blood pressure (NIBP) cuff and the underlying tissue and anatomy when an applied pressure cycle is implemented by the cuff. Therefore, to aid in further elucidating the problems addressed by embodiments of the present invention, certain key findings of the research will now be briefly discussed.

By way of initial background, a clinically standard method of non-invasive blood pressure (NIBP) measurement comprises use of an inflatable cuff which is applied to a body of a patient (typically the upper arm). The cuff is inflated to alter transmural pressure ($P_{tm}$) across the arterial wall of an artery located in the body part beneath the cuff. For example, this may be the brachial artery where the cuff is applied to the upper arm.

Blood pressure oscillations inside the artery cause the artery located under the cuff to oscillate with amplitudes which depend on the $P_{tm}$. Generally, $P_{tm}$ under the cuff is lowered with applied cuff pressure, i.e. $P_{tm} = P_{art} - P_{app}$, where $P_{art}$ is arterial blood pressure inside the artery and $P_{app}$ is the applied pressure by the cuff.

The arterial volume oscillations propagate through arm tissue, generating arm volume pulsations, which in turn generate cuff pressure ($P_{cuff}$) pulsations in the attached cuff. In standard NIBP measurement, the resulting cuff pressure oscillations are processed with known oscillometric algorithms to derive systolic and diastolic blood pressure values, which have been found to be closely related to certain ratios in the acquired oscillation signal.

As discussed previously, accuracy of standard cuff-based NIBP measurements can be unreliable. Furthermore, improvements are difficult to achieve due to the inherent flaws of oscillometry arising from the indirect method of sensing arterial pulsations. In particular, pressure pulsations in the artery transferred into volume pulsations of the artery which are then transferred to pulse propagation through arm tissue, before being transferred into pressure changes in the cuff. Thus, the measurement is indirect, leaving multiple sources for error.

Motivated at least in part by these problems, the inventors have conducted a study using MRI to examine tissue and vasculature behavior under a cuff during measurement across a diverse subject population. Several insights were obtained, pointing to the indirect nature of standard cuff-based NIBP measurement as a main factor in NIBP measurement inaccuracy.

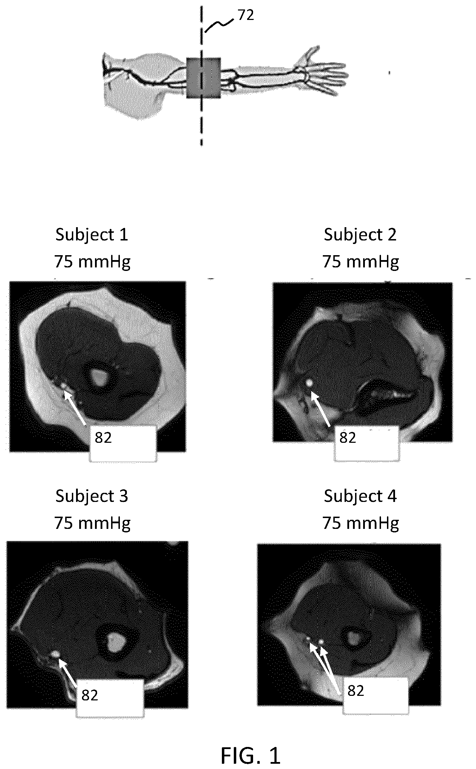

One observation from the study is that the constitution of arm tissue is not homogeneous. A complex, subject-dependent muscle/fat distribution is present. This is illustrated by the sample MRI images of Fig. 1. Each of the images is taken from a different one of four subjects, and represents a cross-sectional slice through the arm along the location of the dashed line 72. The location of the brachial artery 82 is indicated. Each image was obtained while an inflatable cuff is applied to the arm, and wherein the cuff pressure is at 75 mmHg. It can be seen that the fat/muscle thickness and morphology differs for different subjects. This is relevant to blood pressure measurement using a NIBP cuff since the arterial oscillations are transferred to the cuff via the tissue. Thus, the different tissue composition structure for different subjects is a source of inaccuracy.

A further observation is that compression by the cuff is not isotropic. The cuff folds unpredictably during inflation. As a result, pinching of the skin occurs. The applied pressure at the exterior of the arm likely depends upon where the folding occurs with respect to the position of the artery.

A further observation is that the exact artery location is subject-dependent. This can also be seen from Fig. 1, where the location of the brachial artery 82 for each subject is shown. It is likely that oscillations of superficial arteries are expressed differently in the sensed cuff pressure than oscillations of deeper arteries surrounded by subcutaneous fat.

A further observation is that that some subjects present more complex vasculature than others. It is considered likely that this has some effect on the measured cuff pressure signal. For example, in some cases, a subject has two similarly sized brachial arteries, in a branched structure (~ 20% of individuals show such branched brachial arteries), see e.g. Hindawi BioMed Research International Volume 2018, Article ID 1520929. By way of further examples, in some cases the venous component is pulsatile, also affecting measured cuff pressure signal. These variations can impact the obtained signal.

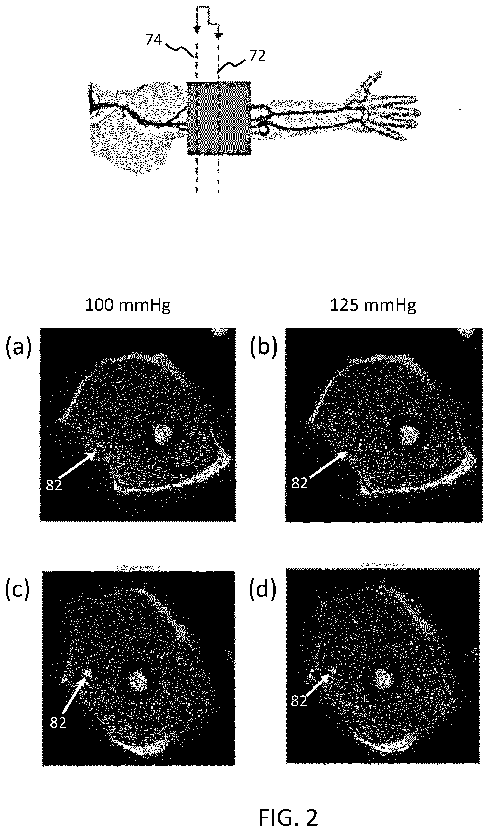

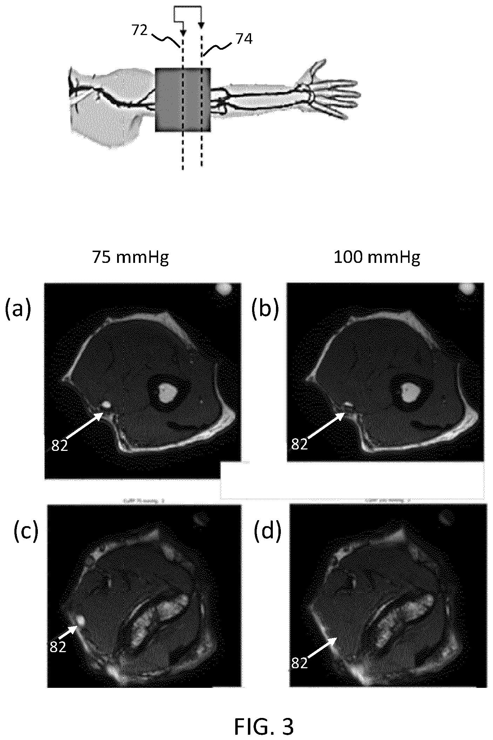

A further observation is that arterial collapse is not uniform across the length of the cuff responsive to pressure applied by the cuff. This is illustrated by the sample MRI images of Fig. 2 and Fig. 3. In each of Fig. 2 and Fig. 3, each of the images represents a cross-sectional slice through the arm of a single subject. The images (a) and (b) in each case represent cross-sectional slices through the arm at a location at the center of the cuff, indicated by dashed line 72. Image (a) was taken with applied cuff pressure at 100 mmHg, and image (b) was taken with applied cuff pressure at 125 mmHg. Images (c) and (d) in each case represent cross-sectional slices through the arm at a location 1 cm offset from an end of the cuff, as indicated by dashed line 74. In Fig. 2, the slices correspond to a location 1cm offset from a proximal end of the cuff, while in Fig. 3 the slices correspond to a location 1cm offset from a distal end of the cuff. In each case, image (c) was taken at an applied cuff pressure of 100 mmHg, while image (d) was taken at an applied cuff pressure of 125 mmHg. In each image the location of the artery 82 is shown.

The images show that the artery collapses at a different rate as a function of cuff pressure at different locations along the length of the cuff. At the center of the cuff (images (a) and (b)), the artery 82 is already partially collapsed at 100 mmHg (image (a)) and is fully collapsed at an applied cuff pressure of 125 mmHg (image (b)). By contrast, for the locations close to the edge of the cuff, the artery is still fully open at an applied cuff pressure of 100 mmHg (image (c)), and only becomes partially collapsed at an applied cuff pressure of 125 mmHg (image (d)). This implies that the cuff pressure is applied less efficiently to tissue at the edges of the cuff compared to at the center of the cuff.

A further observation apparent from the images of Fig. 2 and Fig. 3 is the so-called "knocking effect", or cuff edge effect. This occurs for example as far as 1 cm offset from the proximal end of the cuff. This effect leads to pulsations appearing in the cuff signal even when the artery located under the cuff is completely collapsed. It is therefore difficult to know when supra-systolic cuff pressure has been reached.

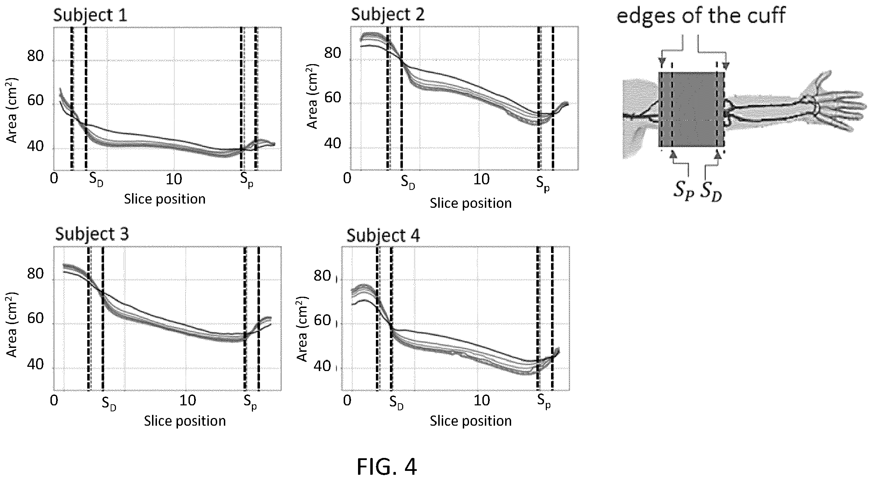

A further observation is that tissue compression dynamics and displacement beneath the cuff involve complex mechanisms which are subject-dependent. From the findings of the inventors, it is unlikely that arterial volume pulsation is equivalent to arm volume pulsation, especially at cuff pressures lower than typical MAP values. This is illustrated for example by Fig. 4 which shows a set of graphs of arm cross-sectional area versus slice position along the longitudinal length of the cuff for four different subjects. Each of graphs (a) to (d) represents measurements for a different subject. The y-axis represents arm cross-sectional area in $cm^2$ and the x-axis represents slice position (in cm) along the length of the cuff and arm. Multiple lines are shown on each graph and these represent measurements for different applied pressures, which it is not possible to fully illustrate due to absence of color in the drawing. Nonetheless, a general pattern can be observed wherein the arm compression at almost all levels of applied pressure differs as a function of position along the arm. In particular, at the edges, the compression by the cuff is different than toward the center. Illustrated in Fig. 4 are dashed line markers corresponding to locations at a proximal end ($S_p$) and distal end ($S_D$) of the cuff respectively. For each subject, at the location toward the distal end of the cuff, arterial compression is less than at the center (arterial area is greater), whereas at the proximal end of the cuff, the arterial compression is greater than at the center (arterial area is less).

Motivated by these observations, embodiments of the present invention provide an alternative approach to measuring blood pressure which aims to at least in part overcome one or more of the above-identified problems.

By way of summary, at least some of the embodiments of the invention aim to overcome uncertainties related to one or more of:

Patient-specific tissue composition.

Patient-specific artery location with respect to cuff.

Non-homogeneous arterial collapse along the length of the cuff.

Effects which occur when two brachial arteries are present.

Cuff edge effects occurring at supra-systolic pressures.

Variable mechanical properties of the cuff.

The non-localized measurement of arterial volume waveform usually acquired via standard NIBP measurement. In standard NIBP measurement, a cuff pressure pulsation is recorded as the pulse travels along the cuff length (e.g., approximately 14 cm). Therefore, the recorded cuff pressure waveform is a distorted version of the original arterial volume waveform. The resulting measured cuff pressure oscillation is difficult to interpret because transmural pressure is not uniform along the length of the cuff. A more localized measure of arterial volume pulsation would be of benefit to more reliably characterize artery area vs. transmural pressure.

Embodiments of the present invention propose to utilize ultrasound imaging to enhance accuracy and reliability of blood pressure measurement. Improved accuracy of inferred blood pressure values is thereby achieved.

By way of summary, one or more embodiments of the present invention may advantageously employ one or more of the following features. An ultrasound transducer array may be used in combination with a pressure actuator, such as an inflatable cuff, and ultrasound image data acquired while an inflation or deflation cycle is implemented. The transducer array may be integrated in the cuff in some examples. The acquired images may be analyzed via image processing methods to segment the artery area. One or more area features may be extracted from the acquired artery area (e. g. maximum and minimum artery areas per pulse, area under the curve of the obtained pulsations, and/or features related to waveform shape). One selected feature or combination of features is then analyzed to quantify changes in artery pulsation as cuff pressure is varied (either via cuff inflation/deflation or a series of applied pressure plateau values). The changes in pulsations with respect to cuff pressure are processed via one or more algorithms to infer blood pressure values.

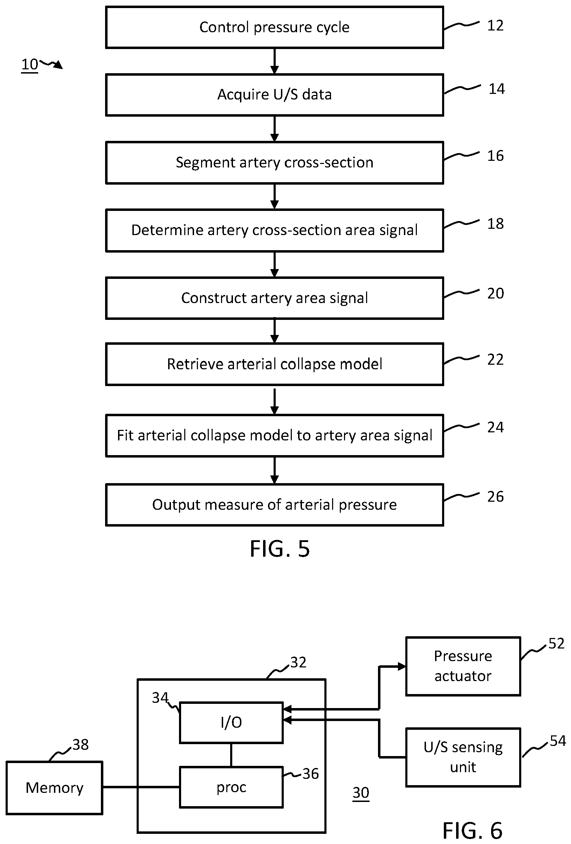

Fig. 5 outlines in block diagram form steps of an example method according to one or more embodiments. The steps will be recited in summary, before being explained further in the form of example embodiments.

The method 10 comprises communicating with and/or controlling a pressure actuator for applying a varying pressure to a body part of a user, and an ultrasound sensing unit for acquiring ultrasound data of an artery in the body part during application of the pressure to the body part.

With regards to the pressure actuator, this may comprise an inflatable cuff for wrapping around a body part of a user in some embodiments, but this is not essential. Any means for applying a controllable compressive pressure to the body part for purposes of compressing an artery located beneath the location of the actuator can be used.

The ultrasound sensing unit may comprise an ultrasound transducer unit. The ultrasound transducer unit may comprise an ultrasound transducer array. The ultrasound sensing unit may further comprise processing modules for processing echo data received at the transducer unit and generating output ultrasound data. The ultrasound data may be ultrasound image data, e.g. B-mode image data, in some examples. The ultrasound data could be sensing data of a different variety or form.

The method more particularly comprises execution of the following steps. These may be computer implemented, for example by a processor or a processing device comprising one or more processors.

The method 10 comprises controlling 12 the pressure actuator to execute a pre-defined applied pressure cycle, wherein an applied pressure to the body part is varied through a range of pressure levels. An applied pressure cycle means a program of applied pressures which are progressed through in a certain sequence and rate. This corresponds for example to the typical process performed in cuff-based NIBP measurement. The pressure cycle may involve starting at a maximum applied pressure and slowly decreasing pressure (e.g. a deflation cycle where an inflatable cuff is used) or may involve starting at a minimum pressure and increasing up to a maximum value (e.g. an inflation cycle).

The method 10 further comprises, during the applied pressure cycle, controlling the ultrasound sensing unit to acquire 14 a time series of ultrasound data frames of the artery spanning the range of applied pressure levels. Although the block scheme of Fig. 5 schematically shows pressure control and the ultrasound acquisition being consecutive, these processes are in fact performed synchronously. Each data frame may for example take the form of an individual ultrasound image, e.g. a B-mode image representative of a cross-sectional plane through the body part. Alternatively, it could comprise non-image ultrasound data, or data at an earlier stage of processing which is suitable to be transformed into an image, e.g. a set of echo data corresponding to a particular time point or corresponding to a particular acquisition event or sequence. The series of data frames spans the applied pressure cycle, meaning that different ultrasound data frames are captured for a series of different applied pressures, for example one for each applied pressure, or one at each of a regular series of pressure intervals along the applied pressure cycle.

The method 10 optionally further comprises applying a segmentation algorithm to the ultrasound data to segment 16 an artery cross-sectional area in each of the series of frames of the ultrasound data. Suitable segmentation algorithms are known in the field. More generally, this step may comprise any method of extracting from each ultrasound data frame a parameter or measure which is indicative of the area, for example a diameter or radius of the artery, or a shortest diametric extension of the artery, or a quantified value of the area itself.

The method 10 may further comprise applying a quantification algorithm to determine 18 a measure indicative of a size of the arterial cross-sectional area in each frame. The quantified value may then be recorded for each data frame in a data array, along with the corresponding applied pressure value of the cuff at which the arterial area value was obtained. The measure may be a measure of the area itself or a measure which is a proxy of area, such as arterial diameter.

The method 10 further comprises constructing 20 an artery area signal indicative of arterial cross-sectional area as a function of applied pressure using the extracted measures from each frame.

The method 10 further comprises retrieving 22 a pre-determined arterial collapse model which relates arterial cross-sectional area, or a measure indicative thereof (e.g. blood vessel diameter or radius), with transmural pressure

($P_{tm}$). In this, transmural pressure may be expressed as a function of applied pressure ($P_{app}$) by the pressure actuator and at least one measure of arterial pressure ($P_{art}$). For example, $P_{tm} = P_{art} - P_{app}$.

The method 10 further comprises fitting 24 the model to the artery area signal. This may comprise fitting parameters of the model to the artery area signal, wherein the parameters of the model include the at least one measure of arterial pressure.

The method 10 may further comprise generating 26 an output indicative of the at least one measure of arterial pressure based on the fitted parameters of the model.

It is noted that the data processing steps 16-26 could be performed synchronously with the ultrasound data acquisition 14 and the controlled pressure cycle 12, or alternatively could be performed during a separate phase after data acquisition, e.g. where the acquired data are recorded in a datastore during the applied pressure cycle for later processing.

As noted above, the method can also be embodied in hardware form, for example in the form of a processing device which is configured to carry out a method in accordance with any example or embodiment described in this document, or in accordance with any claim of this application.

To further aid understanding, Fig. 6 presents a schematic representation of an example processing device 32 configured to execute a method in accordance with one or more embodiments of the invention. The processing device is shown in the context of a blood pressure measurement system 30 which comprises the processing device. The processing device alone represents an aspect of the invention. The blood pressure measurement system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 6, the system 30 further comprises a pressure actuator 52 for applying a varying pressure to a body part of a user. Options for implementing the pressure actuator have been discussed above.

In the illustrated example of Fig. 6, the system 30 further comprises an ultrasound ("U/S") sensing unit 54 for acquiring ultrasound data of an artery in the body part during application of the pressure to the body part. Options for implementing the ultrasound sensing unit 54 have been discussed above.

The system may additionally include a body mount unit adapted to releasably mount to the body of the user for holding the ultrasound sensing unit 54 in a position for acquiring the ultrasound data. In some embodiments, the pressure actuator 52 and the ultrasound sensing unit could be integrated into a common structural support unit, i.e. a common body mount unit. For example, if an inflatable cuff is provided having the ultrasound sensing unit integrated therein, then the cuff may provide the body mount unit, and wherein the actuator is provided by an inflatable bladder integrated in the cuff and the ultrasound sensing unit is carried by the cuff in such a way that, when applied to the body part, the ultrasound transducers are arranged ultrasonically communicable with the artery which is being compressed by the cuff. For example, an ultrasound transducer array could be provided with an ultrasonic input/output area at a surface of the cuff designed to engage with the skin of the user, or at least ultrasonically communicable with such a surface. The ultrasound transducer array may be mounted to a flexible circuit substrate, such as a flexible circuit board.

More explicitly , the processing device 32 may be adapted to: control the pressure actuator to execute a pre-defined applied pressure cycle, wherein an applied pressure to the body part is varied through a range of pressure levels; during the applied pressure cycle, control the ultrasound sensing unit to acquire a time series of ultrasound data frames of the artery spanning the range of applied pressure levels; apply a segmentation algorithm to the ultrasound data to segment an artery cross-sectional area in the series of frames of the ultrasound data; apply a quantification algorithm to determine a measure indicative of a size of the arterial cross-sectional area in each frame; construct an artery area signal indicative of arterial cross-sectional area as a function of applied pressure using the extracted measures from each frame; retrieve a pre-determined arterial collapse model which relates arterial cross-sectional area, or a measure indicative thereof, with transmural pressure, wherein transmural pressure is expressed as a function of applied pressure by the pressure actuator and at least one measure of arterial pressure; fit parameters of the model to the artery area signal, wherein the parameters of the model include the at least one measure of arterial pressure; and generate an output indicative of the at least one measure of arterial pressure based on the fitted parameters of the model.

Although each of the above steps is recited as being performed by a same single processing device 32, in practice, the steps may be performed by one or a plurality of processing components. For example, one processing component might control the applied pressure cycle of the cuff and a separate processing component might perform the operations to process the data and derive the blood pressure measurement.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

As discussed above, a part of the method is obtaining an arterial area signal as a function of applied pressure using a sequence of acquired ultrasound data frames. To further clarify this functionality, an example implementation will now be described.

According to one or more examples, an ultrasound transducer unit of an ultrasound sensing unit is integrated within an inflatable cuff. The inflatable bladder of the cuff provides the pressure actuator and the ultrasound transducer unit is part or the whole of the ultrasound sensing unit. The ultrasound transducer unit is positioned such that the artery area at or toward the center of the cuff is measured during cuff inflation. B-mode images are acquired using data acquired by the ultrasound transducer unit. The transducer unit may at minimum comprise a 1D linear array of ultrasound transducer elements, but more preferably comprises a 2D array. The B-mode images may be generated by processing of echo data acquired at the transducer unit. This processing could be performed by a processing component separate from the cuff, to which the transducer unit is connected via a wired or wireless data connection. B-mode images are suggested as 2D slices through the arm are an effective way of capturing a representation of the artery cross-section. However, another type of imaging could also be considered if it can also be used to acquire a representation of the artery area.

Ultrasound image frames may in some examples be acquired at a frame rate of at least 10 frames per pulse.

For each image, segmentation may be applied to obtain artery area. Many options for segmentation algorithms exist and will be apparent to the skilled person in this field. Instead of segmentation, a different method of extracting a measure representative of arterial area could be considered. For example, machine learning algorithms might be employed which can output a measure of arterial area but without explicitly segmenting the artery cross-section. Thus, more generally, each image frame is processed to extract a measure of arterial cross-sectional area.

The arterial area measures could simply be plotted without further refinement as a function of cuff pressure to thereby derive the plot of arterial area vs cuff pressure. This may then be used as the arterial area signal. This approach is used in some of the examples discussed later in which a model of arterial collapse is fitted to the arterial area signal.

In further cases, the arterial area measures may be computed to extract one or more (preferably a single) representative area value for each pulse, and wherein these representative area values may then be plotted as a function of applied pressure.

With regards to choosing the representative value(s) or features to extract, an aim is that the features are representative of artery pulsation changes due to cuff pressure alteration. These may be plotted as a function of cuff pressure to derive the area signal.

As an example, for each pulse, a minimum area ($A_{min}$) and a maximum area ($A_{max}$) are computed. An upper area envelope signal, $A_{max}$, may be computed as a function of applied cuff pressure. A lower area envelope signal, $A_{min}$, could be computed as a function of applied cuff pressure. A pulse area signal could be computed as ($A_{max}$- $A_{min}$) as function of cuff pressure. Additionally or alternatively, an average area per pulse might also be computed and plotted as a function of cuff pressure. Another possibility is to take the area under the curve of each pulse of the raw area vs cuff pressure plot, and use these values plotted as a function of applied pressure. Any one of these may form the constructed area signal indicative of arterial cross-sectional area as a function of applied pressure.

One benefit of choosing the pulse area signal option ($A_{max}$- $A_{min}$) is that the resulting signal may be directly used to infer blood pressure values because this corresponds to the standard 'envelope' which is extracted during classical oscillometry. Similarly to standard NIBP, empirical ratios may be applied to obtain $P_{sys}$ and $P_{dia}$. For example, according to a common algorithm, the systolic point is found at about 50% of the peak height on the falling phase of the envelope; and the diastolic point is found at about 70% of the peak height on the raising phase of the envelope. These same criteria can be applied to the pulse area signal, and the applied pressure values corresponding to these points can be read off and used as measurements of the systolic and diastolic blood pressure values. The algorithm from which these indicative points are taken is detailed in: the paper: Babbs, C. F. (2012). Oscillometric measurement of systolic and diastolic blood pressures validated in a physiologic mathematical model. Biomedical engineering online, 11(1), 56.

A benefit of ultrasound-based oscillometry is that the ideal ratios will not be influenced by effects stemming from patient-specific tissue characteristics, artery position, edge effects, undefined cuff properties, or artifacts caused travel of pulse along the length of the cuff, therefore enabling more accurate blood pressure measurement.

Other pulse features may be used to compute envelopes representative of artery pulsation changes as response to cuff inflation. One example is area under the curve of the measured pulsations vs. applied pressure. Features related to waveform shape as impacted by applied pressure alteration might also be used. Each envelope may be processed using corresponding optimized ratios to infer blood pressure values.

A variety of technical benefits are achieved through the employment of ultrasound-based sensing of arterial area in blood pressure measurement as compared with standard cuff-based NIBP techniques. At least a subset of the benefits may be summarized as follows:

Patient-specific tissue arm composition, artery location depth, or pulsations generated from multiple arteries of a branching artery structure do not impact artery area measurement accuracy.

No translation of artery volume waveform from a measured cuff pressure waveform is required. Measurement of artery area is direct. This therefore overcomes the difficulty that the exact cuff transfer function is not typically known in standard practice.

While standard NIBP only detects an "additive volume change" as the pulse propagates along the cuff length, ultrasound provides a local measurement of artery oscillation at a single particular location (e.g. the center of the cuff or other suitable location underneath the cuff), therefore overcoming uncertainties related to non-homogeneous arterial collapse along the length of the cuff.

Ultrasound therefore can enable improved estimation of mean arterial pressure (MAP), which is found by identifying cuff pressure at which maximum oscillations occur. Since maximum oscillations are measured specifically at the center of the cuff, there is no interference related to the cuff pressure at which maximum oscillations occur in the other more distal/proximal regions of the cuff.

Uncertainties in pressure envelope characteristics related to cuff edge effects are eliminated, therefore enabling a clear indication of the cuff pressure at which complete arterial collapse is achieved. Arterial pulsations at the edge of the cuff do not influence the measurement of the artery area at the center of the cuff (as opposed to standard oscillometry, where the cuff still detects oscillations even as the artery at the center of the cuff is collapsed).

This also decreases the risk that the cuff is inflated to applied pressure values which are larger than necessary and decreases the required measurement time.

Thus, it can be seen from the preceding descriptions that in fact ultrasound measurements of arterial pulsations can be used to directly infer measures of blood pressure, and that this method confers numerous benefits.

However, according to a preferred set of embodiments of the present invention, it is proposed to further enhance the general approach by utilization of a pre-computed physiological model of arterial collapse as a function of transmural pressure. This allows for more accurate extraction of the values of blood pressure.

In particular, it is proposed in general terms that the measured ultrasound signal is processed through a physiological model of arterial collapse, this replacing the rougher heuristic ratios discussed above. This therefore places less reliance on empirical evidence and is expected to improve the measurement accuracy. Such model-based processing is difficult to achieve through standard NIBP because the cuff and tissue transfer functions, as well as data on the $P_{tm}$ distribution along the length of the cuff, would be needed to accurately measure arterial oscillation amplitude as a function of transmural pressure. In addition, even if tissue and cuff transfer functions were known, the cuff is only capable of measuring relative pulsation amplitudes. This contrasts with ultrasound, which can be used to also acquire absolute artery area and volume.

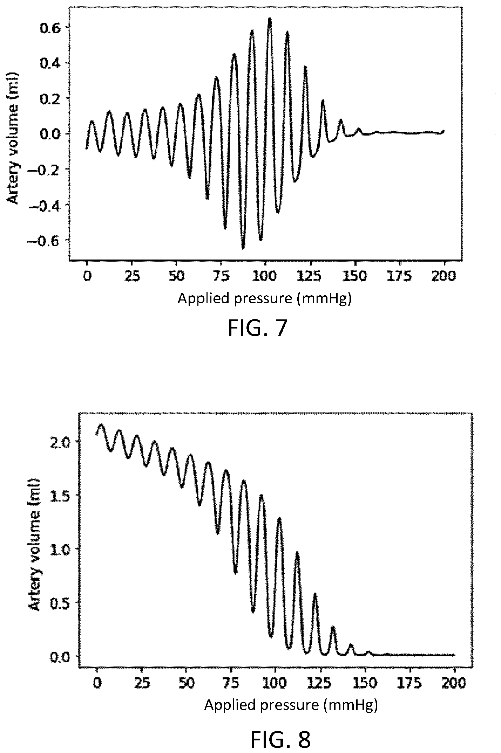

To further illustrate this concept, Fig. 7 and Fig. 8 schematically illustrate this difference between the data obtainable by measuring volume oscillations in a standard NIBP cuff (assuming tissue and cuff transfer functions are known) as a function of applied pressure (Fig. 7), and the data obtainable by directly measuring arterial volume oscillations as a function of applied pressure using ultrasound, where such assumptions are not necessary (Fig. 8). The standard cuff measurements show changes about a baseline, i.e. relative volume values, whereas the ultrasound measurements are able to capture absolute volume oscillations. In these plots, arterial volume is shown as a function of applied pressure. Arterial volume in this case simply corresponds to an instantaneous volume across a length section of the artery, e.g. along a length of the cuff. In other words, it is arterial area multiplied by a length interval, e.g. a length of the cuff. Thus, the arterial volume oscillations can be taken as a proxy for arterial area oscillations. Arterial volume oscillation is a standard way of representing data in oscillometry and thus is used here to aid comparison between the different methods.

With regards to the arterial collapse model which is employed, this in general terms may be a model which reflects expected distortion in the outline shape of the artery wall as compression applied to it by the actuator increases. This distortion leads to a progressive reduction of the artery cross-sectional area as applied pressure increases.

Empirical studies have previously been performed to investigate how arterial area changes as a function of applied pressure, and thus an empirically derived model of arterial collapse exists in the literature and can be represented by the expression:

$$A(P_{tm}) = (d \ln (a P_{tm} + B))/(1 + e^{\wedge}(- c P_{tm}))$$

where A represents blood vessel cross-sectional area, $P_{tm}$ represents transmural pressure across the artery wall, B is a constant, and wherein a, c, and d are tunable parameters of the model, and wherein, when fitting the model, $P_{tm}$ is expressed as a function of applied pressure by the actuator and the at least one measure of arterial pressure, wherein the measure of arterial pressure is a further tunable parameter of the model.

The variable *a* may have units of 1/pressure, e.g. mmHg$^{-1}$. The variable c may have units of 1/pressure, e.g. mmHg$^{-1}$. The variable *d* may have units of distance, e.g. cm. The constant *B* may be a dimensionless constant. By way of example, the value of B may be set at 3.3.

This model is presented by way of example in the following paper: Drzewiecki, G., Hood, R. & Apple, H. Theory of the oscillometric maximum and the systolic and diastolic detection ratios. Ann Biomed Eng 22, 88-96 (1994). Here, a value of B = 3.3 for constant, B, is proposed.

This exact model need not be used, and a different model could instead be employed. A model can be derived either empirically or theoretically.

In general terms, the arterial collapse model may be a model which comprises a ratio of a logarithmic function of transmural pressure and a sigmoid function of transmural pressure. This is for example the general form of the example model expression presented above, and reflects the more general characteristics of arterial collapse as a function of transmural pressure. Thus, any model represented by a function having this form should be sufficient.

Various example implementations of the particular example arterial collapse model outlined above will now be discussed, including fitting the model and the method for extracting quantitative measures of blood pressure from the model.

Considering the model expression outlined previously, area is represented as a function of transmural pressure. Transmural pressure can of course be represented as a sum of arterial pressure and externally applied pressure by the actuator. Usually the applied pressure is subtracted from the arterial pressure, such that transmural pressure, $P_{tm}$ can be represented as

$$P_{tm} = P_{art} - P_{app}$$

where $P_{art}$ is instantaneous arterial pressure inside the artery, and $P_{app}$ is the applied pressure from the pressure actuator (e.g. cuff).

The model function may be thus re-expressed with $P_{tm}$ expressed in terms of applied pressure and arterial pressure:

$$A = (d \ln (a (P_{art} - P_{app}) + B))/(1 + e^{-c (P_{art} - P_{app})})$$

where A represents blood vessel cross-sectional area, $P_{tm}$ represents transmural pressure, $P_{app}$ represents applied pressure by the actuator, $P_{art}$ represents (instantaneous) arterial blood pressure, and B is a constant.

It is proposed to fit this model to the acquired arterial area signal in order thereby to infer at least one measure of arterial pressure.

In its form outlined above, the variable representing arterial pressure, $P_{art}$ is instantaneous arterial pressure. It is more convenient to represent this as a function of one of diastolic pressure, $P_{dia}$, or systolic pressure, $P_{sys}$, for purposes of fitting the model, and then to use $P_{dia}$ or $P_{sys}$ as one of the independent variables of the model fitting. In this way a value for $P_{dia}$ or $P_{sys}$ can be derived from simply reading off the fitted value of the variable once the model has been optimally fit.

Thus, for example, when performing the model fitting, $P_{art}$ may be expressed as a function of $P_{dia}$ or $P_{sys}$ and a sinusoidal function. For example, $P_{art}$ may be expressed as $P_{art} = (P_{dia} + \Gamma \sin \omega t)$, where $\Gamma$ represents pulse pressure (which is the difference between systolic and diastolic pressure of a single pulse) $\omega$ represents heart or pulse rate, $t$ represents time of each arterial area measurement, and wherein each of $P_{dia}$, $\Gamma$, $\omega$, a, c and d are tuneable parameters . Since $t$ is a second independent variable, fitting the model could be done by multiple regression fitting. Alternatively, since $P_{app}$ is in practice a function of time, then, when fitting the model $P_{art}$ might be expressed instead as: $P_{art} = (P_{dia} + \Gamma \sin \omega P_{app})$.

Here, the measure of arterial pressure to be derived from the model fitting is $P_{dia}$ and is obtained by simply reading off the fitted value for the $P_{dia}$ parameter in the model fitting. A value for $P_{sys}$ may then be derived by simply adding the fitted value for $\Gamma$ onto the fitted valued for $P_{dia}$.

Alternatively, when fitting the model, $P_{art}$ may be expressed as $P_{art} = (P_{sys} - \Gamma \sin \omega t)$ or as $P_{art} = (P_{sys} + \Gamma \sin \omega P_{app})$, where $\Gamma$ represents pulse pressure (which is the difference between systolic and diastolic pressure), t represents time of each arterial area measurement, and $\omega$ represents heart or pulse rate, and wherein each of $P_{sys}$, $\Gamma$, $\omega$, a, c and d are tuneable parameters. Here, the measure of arterial pressure to be derived from the model fitting is $P_{sys}$, and is obtained by simply reading off the fitted value for the $P_{sys}$ parameter in the model fitting. A value for $P_{dia}$ may then be derived by simply adding the fitted value for $\Gamma$ onto the fitted valued for $P_{sys}$.

For fitting the model, a regression fitting method can be used. The arterial area signal comprises a data series of data points, where each data point comprises a value of arterial cross-sectional area and a corresponding value of applied pressure by the actuator, $P_{cuff}$. The remaining parameter can then be tuned for example by adjusting values of the tuneable parameters of the model so as to minimise the optimisation function:

$$minimise \sum_{P\_app=0}^{p\_app=pmax} \left[A_{obs}(P_{app}) - A_{model}(P_{app}, \beta)\right]^2$$

where $A_{obs}$ is the ultrasound-measured area signal for the artery, $P_{app}$ is applied pressure by the actuator, and $A_{model}$ is the fitted model, where $\beta$ represents collectively all of the independently tuneable parameters of the model.

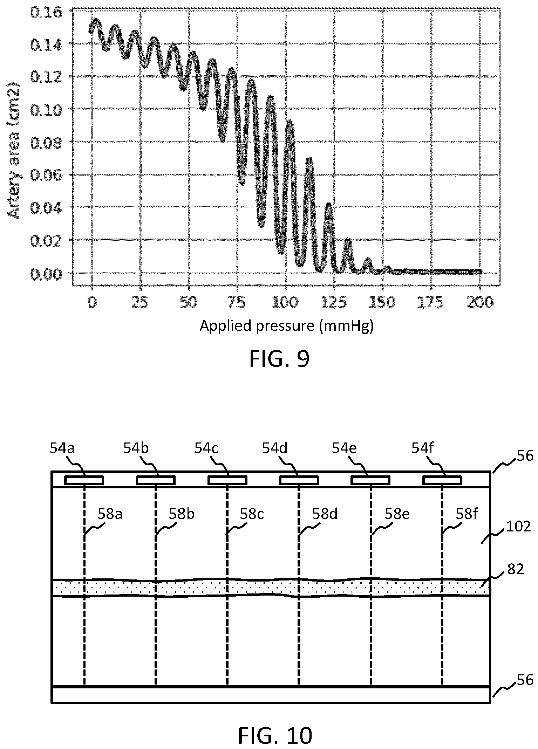

Fig. 9 illustrates an example of a fitted model area function. The model fits very well to the original measured area signal.

As an alternative to representing $P_{art}$ in terms of a sinusoidal function, the model fitting could be simplified by first processing the arterial area signal to extract either an upper or lower envelope of the arterial area signal, and then fitting the arterial collapse model to the upper or lower envelope. Thus, here, the arterial area signal starts as a plot of the raw obtained arterial area values as a function of cuff pressure. Then, an upper envelope, $A_{env-up}(P_{app})$, and/or a lower envelope $A_{env-low}(P_{app})$ is extracted, and the arterial collapse model outlined previously is fit to one of these area envelope signals.

Thus, for example, for the fitting, the arterial collapse model may take the form:

$$A_{env-up} = d \ ln(a \ (P_{sys} - P_{app}) + B)/ (1 + exp(-c(P_{sys} - P_{app})))$$

where $A_{env-up}$ represents the upper envelope of the blood vessel cross-sectional area signal, $P_{sys}$ represents systolic blood pressure, $P_{app}$ represents applied pressure by the actuator, and B is a constant, and wherein each of $P_{sys}$, a, c and d are tuneable parameters. The measure of arterial pressure derived in this case is systolic blood pressure, $P_{sys}$, and is obtained by simply reading off the fitted value of the variable $P_{sys}$.

Alternatively, for the fitting, the arterial collapse model make take the form:

$$A_{env-low} = d \ ln(a \ (P_{dia} - P_{app}) + 3.3)/ (1 + exp(-c(P_{dia} - P_{app})))$$

where $A_{env-low}$ represents the lower envelope of the blood vessel cross-sectional area signal, $P_{dia}$ represents diastolic blood pressure, and $P_{app}$ represents applied pressure by the actuator, and B is a constant, and wherein each of $P_{dia}$, a, c and d are tuneable parameters. The measure of arterial pressure derived in this case is diastolic blood pressure, $P_{dia}$, and is obtained by simply reading off the fitted value of the variable $P_{dia}$

In some embodiments, both the upper and lower envelopes may be separately fitted, to yield values for both $P_{sys}$ and $P_{dia}$. Thus, in some embodiments, fitting the model may comprise a first fitting operation and a second fitting operation, wherein the first fitting operation comprises fitting the upper envelope of the measured arterial area signal to the model of arterial collapse and the second fitting operation comprises fitting the lower envelope of the measured arterial area signal to the model of arterial collapse.

In some embodiments, once the arterial area vs transmural pressure model has been fitted to the acquired area signal, and the measure of arterial pressure derived from the fitting parameters, the method may further comprise, without applying any cuff pressure, continuing to acquire ultrasound data of the same blood vessel, monitoring changes in arterial area using ultrasound data; using the model of area vs transmural pressure to map changes in area to inferred changes in transmural pressure; and using the changes in transmural pressure as a proxy for changes in arterial blood pressure. In this way, a real-time or continuous arterial blood pressure signal can be obtained by tracking changes in inferred transmural pressure over time via tracking changes in arterial area.

In some embodiments, a measure indicative of arterial compliance could additionally be obtained from the fitted model of arterial area vs transmural pressure. In particular, arterial compliance is defined as artery area (or volume) change divided by transmural pressure change. Thus, in other words, the general slope or gradient of the fitted area vs transmural pressure model provides an indication of arterial compliance. Both arterial compliance and arterial blood pressure values may therefore be derived, and these two measures together provide a more powerful predictive indicator than blood pressure alone. For example, a low arterial compliance twinned with a low blood pressure for example can be indicative of sepsis.

In accordance with some embodiments, it is proposed to acquire area measurements at multiple different locations along the length of the artery beneath the actuator. This may enable a more accurate measure of blood pressure by taking into account possible variations in the pressure transfer from the actuator to the body part.

In particular, as discussed above with reference to Fig. 4, a finding of the inventors is that arterial collapse is not uniform along the length of a compressive cuff. Transmural pressure at the proximal end region of the cuff is higher than transmural pressure at the center part of the cuff. The opposite happens distally: transmural pressure at the center of the cuff is higher than transmural pressure at the distal end part of the cuff. It is a hypothesis of the inventors that these effects may be caused by a combination of non-uniform pressure distribution along the artery, complex tissue compression, displacement and folding and differences in internal blood pressure proximally vs. distally with respect to the cuff. The latter effect takes place due to the compressed artery and vein cutting circulation in/out of the limb. During the occlusion time, distal venous and arterial pressures tend towards an equilibrium value . As the cuff deflation starts, complex mechanisms occur. Blood flow in and out of the arm resumes but with a distal arterial pressure that is lower than usual and a venous pressure that is higher than usual. Reference is made for example to the following paper which discusses some of these phenomena in more depth: Rodríguez-Niedenführ M, Vázquez T, Neam L, Ferreira B, Parkin I, Sañudo JR. Variations of the arterial pattern in the upper limb revisited: a morphological and statistical study, with a review of the literature. J Anat. 2001 Nov; 199(Pt 5):547-66. PMID: 11760886.

Thus, according to one or more embodiments, the processing device may be configured, during the applied pressure cycle, to acquire a respective time series of ultrasound data frames for each of a plurality of spatial planes through the artery, wherein the plurality of spatial planes are spaced from one another along a direction of a longitudinal length of the artery. These could be acquired using a single ultrasound transducer unit or by using multiple ultrasound transducer units, each located at a different respective position along the length of the cuff.

An example is illustrated schematically in Fig. 10 which shows an example cuff 56 integrating an inflatable bladder, and which is shown applied to an arm 102 of a subject. The brachial artery location 82 is indicated. The cuff integrates a plurality of spaced ultrasound transducer units 54a, 54b, 54c, 54d, 54e, 54f. In this example, each ultrasound transducer unit comprises a transducer array. The ultrasound transducer units are spaced from one another along the longitudinal (axial) dimension of the cuff, when the cuff is rolled around the arm. The set of ultrasound transducer units each is arranged to acquire ultrasound data representative of a respective spatial plane 58a, 58b, 58c, 58d, 58e, 58f. Each spatial plane extends obliquely with respect to the longitudinal axis of the cuff and of the arm, for example normal to the longitudinal axis. Each represents a respective cross-section across the arm. A respective time series of ultrasound data frames is acquired for each of the plurality of spatial planes through the artery. The whole set of ultrasound transducer units may be comprised by a single ultrasound sensing unit which collects the data acquired by each and transfers it to the processing device 32, or each may form part of a separate ultrasound sensing unit.

It is noted that although the schematic diagram of Fig. 10 shows data for six spatial planes being acquired, the exact number may differ. There may be acquired data for a greater number or a fewer number of spatial planes.

The data for the multiple locations along the artery can be used in a variety of different ways.

In one set of embodiments, it is proposed to use the extra data to improve the deriving of the blood pressure measures. For example, the processor may be adapted to construct an artery area signal as a function of time for each of the spatial planes 58a-58f, and wherein the arterial collapse model is fitted to the respective artery area signal for each of the spatial planes. The multiple fitted area signals may be used to compute an aggregated area signal, for example an average area signal, for example a mean or median area signal. A preferred example would be to compute a weighted average arterial area signal, wherein each area signal in the sum is weighted according to estimated non-physiological area variation at that position along the length of the cuff.

The model fitting discussed above may then be performed for this aggregated version of the area signal. This may improve the accuracy of the derived blood pressure measures.

Additionally or alternatively, multi-location ultrasound may be used together with an active, multicompartment cuff to apply pressure such that artery area is uniform along the length of the cuff.

To state this more generally, where the system comprises a cuff for attachment to an arm of the patient, and the cuff comprises the pressure actuator, the actuator may comprise a plurality of actuator elements, each element arranged for applying pressure to the artery across a different respective section of the length of the cuff, and each having an independently controllable applied pressure. For example, the cuff, when worn, has a length for extending along a longitudinal direction of the artery.

Here, the plurality of spatial planes 58 referred to previously may include at least one spatial plane aligned longitudinally within each of the length sections of the cuff. The processing device 32 may then be configured to control the applied pressures of the plurality of actuator elements such that an artery area measured within each length section at any given time is the same. For example, the different actuator elements might take the form of different independently inflatable compartments of an inflatable bladder.

Additionally or alternatively, in accordance with one or more embodiments, the multi-location ultrasound may be used to measure tissue longitudinal displacement, which is known to be an important factor interfering with pressure transfer from the actuator to the arterial wall. In this regard, reference is made for example to the following paper: Alexander H, Cohen ML, Steinfeld L. Criteria in the choice of an occluding cuff for the indirect measurement blood pressure. Med Biol Eng Comput. 1977 Jan;15(1):2-10. Longitudinal tissue displacement was also independently observed by the inventors in the MRI study referenced previously. For example, reference is made to Fig. 4 which shows that complex compression and displacement effects occur along the length of the cuff.

In some embodiments, where ultrasound data is acquired for each of a plurality of spatial planes at spaced longitudinal locations (as discussed above), it is proposed to analyze the time series of ultrasound data frames for each of the plurality of spatial planes through the artery to compute a measure of longitudinal tissue displacement at a longitudinal location of each of the spatial planes. For example, this can be achieved by acquiring a time series of image frames for each of the planes, and then detecting tissue motion based on tracking changes in tissue appearance across time for each location. Preferably, the images at each location can be used to identify the region around the center of the cuff over which displacement does not occur or occurs the least. In this case, only this identified arterial segment would be taken into account for blood pressure estimation. In other words, it is proposed to identify the one of the spatial planes for which longitudinal tissue motion is minimum, and perform the fitting of the arterial collapse model and the computation of the at least one measure of arterial pressure using the ultrasound data from the location of said identified one of the spatial planes.

Alternatively, a model may be used to correct for the tissue displacement. For example a model may be used which allows for mapping between measured longitudinal tissue displacement at a certain location and an estimated additional pressure term to subtract or add to the transmural pressure value for each location. For example, reference is made to the following paper from which a suitable model may be derived: Alexander H, Cohen ML, Steinfeld L. Criteria in the choice of an occluding cuff for the indirect measurement blood pressure. Med Biol Eng Comput. 1977 Jan;15(1):2-10. doi: 10.1007/BF02441568. PMID: 194119.

Another way in which a multiplicity of arterial area measurements could be used is to supplement an additional, different method of inferring blood pressure values previously proposed by the Applicant, and which utilizes measured pulse arrival time (PAT) values to infer values for blood pressure. The relevant method is detailed in the document: EP4173556A1. PAT is well known as a reliable surrogate for arterial blood pressure. Pulse arrival time is defined as the time interval between the peak of the R wave of the electrocardiogram (ECG) and the onset of a peripheral plethysmographic pulse (i.e. detection of the corresponding pulse at a peripheral arterial site, e.g. by a PPG sensor). Physiologically, PAT is the sum of the pre-ejection period (PEP) and the pulse transit time (PTT).

The method of EP4173556A1 employs a pre-determined transfer function of PAT as a function of a measure of arterial pressure, such that by measuring PAT, the measure of arterial pressure can be inferred. The transfer function between PAT and the measure of arterial pressure has a dependency upon a calibration factor. Acquisition of the calibration factor involves measuring PAT values at a peripheral location as a function of changing applied cuff pressure values of an NIBP cuff. As explained in paragraphs [0054]-[0057] of EP4173556A1, the PAT vs arterial blood pressure model would ideally be regression fitted using multiple pairs of PAT and arterial blood pressure values. However, such data is typically not available or would not be possible to acquire over a short period of time. Arterial blood pressure of an individual can be expected to remain roughly steady over a short period of time, and thus obtaining such data pairs over a broad range of different arterial pressure values in a short space of time is not possible. As explained at paragraph [0057] of EP4173556A1, instead, changing transmural pressure caused by changing applied cuff pressure can be used as a surrogate for changing arterial blood pressure. Thus, by measuring PAT values over the course of a varying applied pressure cycle by an NIBP cuff, calibration for the PAT vs arterial pressure model can be achieved.

In particular, the method of EP4173556A1 comprises executing a standard NIBP cuff measurement cycle (involving varying the applied pressure exerted by the cuff). The applied pressure values by the cuff are recorded. PAT values over the course of the measurement cycle are recorded. The arterial blood pressure (BP) values measured by the NIBP measurement cycle (e.g. one pair of systolic and diastolic pressure values) are recorded. From this, change in PAT as a function of change in transmural pressure is derivable. This provides the calibration of the PAT vs arterial pressure model or transfer function.

In particular, as explained in paragraph [0058] of EP4173556A1, using obtained pairs of PATs, the synchronously acquired cuff pressure and the BP values, a PAT/BP sensitivity parameter S can be derived, which enables to track BP. This is illustrated in Fig. 5 of EP4173556A1 showing a schematic diagram illustrating the general concept of calibration. During cuff inflation, the increasing applied pressure causes a PAT increase to be induced, from which, together with the synchronously acquired cuff pressure and the derived BP (via oscillometry), a sensitivity parameter S is derived to track BP. In other words, during calibration, S is derived as change in measured PAT as a function of induced change in transmural pressure. Subsequently, during BP inference, S is used as a multiplier to map measured PAT values to arterial blood pressure values. Optionally, a subject-specific scaling factor, *k,* can also be used in the transfer function. A non-limiting example model or transfer function using the inferred sensitivity parameter S which may be used to infer BP from measured PAT valued is reproduced below:

$$BP(t) = k \cdot S \cdot (PAT - PAT_0) + BP(t0)$$

where BP(t) represents real-time inferred arterial pressure, k represents a subject specific scaling factor, S represents the sensitivity parameter (the calibration parameter) measured during calibration, PAT represents real time measured PAT value, $PAT_0$ represents PAT at a start of the measurement period, and BP(t0) represents arterial blood pressure as measured once using an NIBP cuff at a start of the measurement period.

The reader is referred to EP4173556A1 for more comprehensive details regarding this non-invasive BP inference method.

It is the realization of the inventors that the new concepts employed in accordance with the present invention may be advantageously applied to improve the PAT-based blood pressure inference method.

In particular, it is a limitation of the existing method that transmural pressure at the cuff side is not well-defined. In particular, the method of EP4173556A1 assumes that there is a uniform transmural pressure change induced by the applied cuff along the whole length of the cuff. However, as has been discussed above, this assumption is not fully correct.

It is proposed in accordance with one set of embodiments to use the previously discussed arterial collapse model to infer a transmural pressure vs time signal for each of a plurality of longitudinal locations along the cuff.

In particular, this is based on the observation of the inventors that the arterial area vs transmural pressure relationship can be assumed to be the same along the length of the cuff (this being physiologically determined). Ultrasound may be used to assess the artery area vs actuator applied pressure along the length of the cuff during cuff inflation, therefore estimating what transmural pressure is applied at each point along the cuff length. This can be used to more correctly interpret the PAT change which occurs as response to a transmural pressure change. For example, a measured PAT change might be observed to occur as an additive response to a $P_{tm}$ change of, e.g. 10 mmHg at a center/distal region of the cuff and a $P_{tm}$ change of, e.g. 5 mmHg at a proximal region of the cuff.

Thus, in some embodiments, an operation is proposed wherein the multiple area signals acquired for the multiple spatial planes 58 at the different longitudinal locations (as discussed above) are used to infer a corrected function of transmural pressure vs time at each plane location based on an assumption that measurements at a most central location are the most accurate. The calibration factor can be computed based on measured change in PAT vs change in the more accurate measure of transmural pressure at each location.

In particular, in some embodiments, the processing device may be adapted to construct an artery area signal as a function of time for each of the aforementioned spatial planes. The system may comprise a cuff for attachment to an arm (or other body part) of the patient, wherein the cuff comprises the pressure actuator, and wherein the cuff, when worn, has a length for extending along a longitudinal direction of the artery, and wherein the applied pressure is applied along at least a section of the length of the cuff. The processing device may be configured to fit the arterial collapse model to a first of the plurality of artery area signals, for example wherein the first corresponds to a central location along the length of the cuff, to derive a first fitted model. The processing device may be further adapted to use the first fitted model to infer a transmural pressure vs time signal for the locations of each of the other of the plurality of spatial planes, based on the respective artery area signal for each spatial plane, and based on an assumption that the same fitted model applies to each location, to thereby derive a respective transmural pressure vs time signal for each of the locations of the plurality of spatial planes.

Here, the assumption is that the transmural pressure is distorted at edge regions of the cuff due to physical inhomogeneity of pressure transfer to tissue from different regions of the cuff. This is based on empirical findings of the inventors, as discussed above (see e.g. Fig. 4 and accompanying discussion).

It is therefore proposed to fit the model for a central location. The findings of the inventors show that the central location has the least distortion in the pressure transfer between cuff and blood vessel. From this, transmural pressure as a function of time can be inferred at the other locations by assuming that the central fitted model is correct and that the area measurements at the other locations are correct.

In some embodiments, the processing device may be further adapted to retrieve a model of a measure of arterial pressure as a function of PAT, wherein the model relates the measure of arterial pressure PAT as a function of a calibration factor. The processing device may be further adapted to compute a value of the calibration factor based on the aforementioned plurality of transmural pressure vs time signals for the locations of the plurality of spatial planes along with a synchronously acquired PAT vs time signal. Pairs of time-matched PAT and transmural pressure values can be used to derive the calibration factor.

For example, an aggregated (e.g. average) calibration factor might be computed based on the transmural pressure vs time signals for the plurality of locations. Either an average transmural pressure vs time signal could be first computed for the plurality of longitudinal locations and the calibration factor derived from this, or a plurality of calibration factors computed and an average taken from these. The processing device may further be adapted to: receive a real-time PPG signal and a real-time ECG signal; compute a real-time PAT signal based on the PPG signal and ECG signal; and use the model the measure of arterial pressure as a function of PAT to convert the PAT signal to a real-time arterial blood pressure signal. For example, the PPG signal may be received from a PPG sensor attached at a peripheral arterial site of the subject. For instance, if the cuff is attached to the upper left arm, the PPG sensor might be arranged to measure a PPG signal at the left hand or a finger of the left hand.

By way of example, one suitable model of arterial pressure as a function of PAT has been outlined above, wherein S represents the calibration parameter.

Additionally or alternatively to the embodiment described above for deriving a calibration factor based on inferred transmural pressure at multiple locations, in some embodiments, the blood pressure inference method of EP4173556A1 could be applied in combination with the previously described embodiment in which multi-location ultrasound is used together with an active, multicompartment cuff to apply pressure such that the artery area is uniform along the length of the cuff. In particular, the system may comprise a cuff for attachment to an arm of the patient, the cuff comprising a pressure actuator, and wherein the actuator comprises a plurality of actuator elements, each element arranged for applying pressure to the artery across a different respective section of the length of the cuff, and each having an independently controllable applied pressure. For example, the cuff, when worn, has a length for extending along a longitudinal direction of the artery. The processing device 32 may then be configured to control the applied pressures of the plurality of actuator elements such that an artery area measured within each length section at any given time is the same. For example, the different actuator elements might take the form of different independently inflatable compartments of an inflatable bladder. In this case, a single calibration factor could be computed based on measurements acquired at a single location along the cuff since it is known that the transmural pressure is uniform along the length of the cuff.

Further optional features which may be implemented in accordance with any embodiment described in this disclosure will now be discussed.

In some embodiments, it is proposed to record ultrasound data representing each of two or more branches of an artery in the user's body part. A respective arterial area signal could be derived for each and these might be fused or aggregated into a single combined arterial area signal. This fused area signal could then be used to derive the arterial pressure measure using techniques already discussed above. The fusing of the area signals could be performed using an averaging of the signals, for example a weighted averaging. The weights could be determined based on properties (e.g. size) of the observed arteries and/or their location in the arm.

In some embodiments, it is proposed to use the obtained arterial area signal to derive a measure of cuff-to-artery transfer pressure properties. This may have particular value in the case where multiple arterial area signals are acquired at different locations along the length of the cuff. Here, variations in pressure transfer properties along the length of the cuff can be inferred.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A blood pressure measurement system (30), comprising:

   a pressure actuator (52) for applying a varying pressure to a body part of a user;
   an ultrasound sensing unit (54) for acquiring ultrasound data of an artery in the body part during application of the pressure to the body part;
   a processing device (32) adapted to:

   control the pressure actuator to execute a pre-defined applied pressure cycle, wherein an applied pressure to the body part is varied through a range of pressure levels;
   during the applied pressure cycle, control the ultrasound sensing unit to acquire a time series of ultrasound data frames of the artery spanning the range of applied pressure levels;
   apply a quantification algorithm to determine a measure indicative of a size of an arterial cross-sectional area in each frame;
   construct an artery area signal indicative of arterial cross-sectional area as a function of applied pressure using the extracted measures from each frame;
   retrieve a pre-determined arterial collapse model which relates arterial cross-sectional area, or a measure indicative thereof, with transmural pressure, wherein transmural pressure is expressed as a function of applied pressure by the pressure actuator and at least one measure of arterial pressure;
   fit parameters of the model to the artery area signal, wherein the parameters of the model include the at least one measure of arterial pressure;
   generate an output indicative of the at least one measure of arterial pressure based on the fitted parameters of the model.

2. The system of claim 1, wherein the arterial collapse model comprises a ratio of a logarithmic function of transmural pressure and a sigmoid function of transmural pressure.

3. The system of claim 1 or 2, wherein the arterial collapse model is represented by the expression:

$$A(P_{tm}) = (d \ln(a\,P_{tm} + B))/(1 + e^{\wedge}(-c\,P_{tm}))$$

   where A represents blood vessel cross-sectional area, $P_{tm}$ represents transmural pressure, B is a constant, and wherein a, c, and d are tunable parameters, and wherein, when fitting the model, $P_{tm}$ is expressed as a function of applied pressure by the actuator and the at least one measure of arterial pressure, wherein the measure of arterial pressure is a further tunable parameter.

4. The system of claim 3, wherein, when fitting the model, the arterial collapse model is expressed as:

$$A = (d \ln(a\,(P_{art} - P_{app}) + B))/(1 + e^{-c\,(P_{art} - P_{app})})$$

   where A represents blood vessel cross-sectional area, $P_{art}$ represents instantaneous arterial blood pressure, $P_{app}$ represents applied pressure by the actuator, and B is a constant,

   and wherein, when fitting the model fitting, $P_{art}$ is expressed as $P_{art} = (P_{dia} + \Gamma \sin \omega t)$, where $\Gamma$ represents pulse pressure, $\omega$ represents heart rate, and $t$ represents time, and
   and wherein the measure of arterial pressure is $P_{dia}$,
   and wherein each of $P_{dia}$, $\Gamma$, $\omega$, a, c and d are tuneable parameters.

5. The system of any of claims 1-3,

   wherein the fitting the model comprises extracting an upper envelope of the measured arterial area signal, and fitting the upper envelope of the measured arterial area signal to the model of arterial collapse,
   wherein, for the fitting, the arterial collapse model takes the form:

$$A_{env-up} = d\ ln(a\ (P_{sys} - P_{app}) + B)/(1 + exp(-c(P_{sys} - P_{app})))$$

where $A_{env-up}$ represents the upper envelope of the blood vessel cross-sectional area signal, $P_{sys}$ represents systolic blood pressure, $P_{app}$ represents applied pressure by the actuator, and B is a constant;
and wherein the measure of arterial pressure is systolic blood pressure, $P_{sys}$,
and wherein each of $P_{sys}$, a, c and d are tuneable parameters.

6. The system of any of claims 1-3 or claim 5,
wherein the fitting the model comprises extracting a lower envelope of the measured arterial area signal, and fitting the lower envelope of the measured arterial area signal to the model of arterial collapse,

wherein, for the fitting, the arterial collapse model takes the form:

$$A_{env-low} = d\ ln(a\ (P_{dia} - P_{app}) + B)/(1 + exp(-c(P_{dia} - P_{app})))$$

where $A_{env-low}$ represents the lower envelope of the blood vessel cross-sectional area signal, $P_{dia}$ represents diastolic blood pressure, and $P_{app}$ represents applied pressure by the actuator, and B is a constant;
and wherein the measure of arterial pressure is diastolic blood pressure, $P_{dia}$,
and wherein each of $P_{dia}$, a, c and d are tuneable parameters.

7. The system of any preceding claim, wherein the processing device is adapted, during the applied pressure cycle, to acquire a respective time series of ultrasound data frames for each of a plurality of spatial planes through the artery, wherein the plurality of spatial planes are spaced from one another along a direction of a longitudinal length of the artery.

8. The system of claim 7, wherein the processor is adapted to construct an artery area signal as a function of time for each of the spatial planes, and wherein the arterial collapse model is fitted to the respective artery area signal for each of the spatial planes.

9. The system of claim 7,

wherein the processing device is adapted to construct an artery area signal as a function of time for each of the spatial planes;
wherein the system comprises a cuff for attachment to an arm of the patient, wherein the cuff comprises the pressure actuator, wherein the cuff, when worn, has a length for extending along a longitudinal direction of the artery, and wherein the applied pressure is applied along at least a section of the length of the cuff;
wherein the processing device is adapted to fit the arterial collapse model to a first of the plurality of artery area signals, to derive a first fitted model;
wherein the processing device is further adapted to use the first fitted model to infer a transmural pressure vs time signal for the locations of each of the other of the plurality of spatial planes, based on the respective artery area signal for each spatial plane, and based on an assumption that the same fitted model applies to each location, to thereby derive a respective transmural pressure vs time signal for each of the locations of the plurality of spatial planes.

10. The system of claim 9,

wherein the processing device is further adapted to retrieve a model of a measure of arterial pressure as a function of pulse arrival time (PAT), wherein the model relates the measure of arterial pressure to PAT as a function of a calibration factor;
wherein the processing device is further adapted to compute a value of the calibration factor based on the plurality of transmural pressure vs time signals for the locations of the plurality of spatial planes;
wherein the processing device is further adapted to:

receive a real-time PPG signal and a real-time ECG signal;
compute a real-time PAT signal based on the PPG signal and ECG signal;
use the model of arterial pressure as a function of PAT to convert the PAT signal to a real-time arterial blood pressure signal.

**11.** The system of any of claims 7-10,

wherein the system comprises a cuff for attachment to an arm of the patient, wherein the cuff comprises the pressure actuator, wherein the cuff, when worn, has a length for extending along a longitudinal direction of the artery, and wherein the applied pressure is applied along at least a section of the length of the cuff; and wherein the actuator comprises a plurality of actuator elements, each element arranged for applying pressure to the artery across a different respective section of the length of the cuff, and each having an independently controllable applied pressure.

**12.** The system of claim 11,

wherein the plurality of spatial planes includes at least one spatial plane aligned longitudinally within each of the length sections of the cuff; and wherein the processing device is adapted to control the applied pressures of the plurality of actuator elements such that an artery area measured within each length section at any given time is the same.

**13.** The system of any claims 7-12, wherein the processing device is adapted to:

analyze the time series of ultrasound data frames for each of the plurality of spatial planes through the artery to compute a measure of longitudinal tissue displacement at a longitudinal location of each of the spatial planes; identify the one of the spatial planes for which longitudinal tissue motion is minimum; perform the fitting of the arterial collapse model and the computation of the at least one measure of arterial pressure using the ultrasound data from the location of said identified one of the spatial planes.

**14.** A blood pressure measurement method (10),

the method comprising communicating with:

a pressure actuator (52) for applying a varying pressure to a body part of a user, and an ultrasound sensing unit (54) for acquiring ultrasound data of an artery in the body part during application of the pressure to the body part;

the method comprising:

controlling (12) the pressure actuator to execute a pre-defined applied pressure cycle, wherein an applied pressure to the body part is varied through a range of pressure levels; during the applied pressure cycle, controlling the ultrasound sensing unit to acquire (14) a time series of ultrasound data frames of the artery spanning the range of applied pressure levels; applying (18) a quantification algorithm to determine a measure indicative of a size of an arterial cross-sectional area in each frame; constructing (20) an artery area signal indicative of arterial cross-sectional area as a function of applied pressure using the extracted measures from each frame; retrieving (22) a pre-determined arterial collapse model which relates arterial cross-sectional area, or a measure indicative thereof, with transmural pressure, wherein transmural pressure is expressed as a function of applied pressure by the pressure actuator and at least one measure of arterial pressure; fitting (24) parameters of the model to the artery area signal, wherein the parameters of the model include the at least one measure of arterial pressure; generating (26) an output indicative of the at least one measure of arterial pressure based on the fitted parameters of the model.

**15.** A computer program product comprising computer program code configured to cause execution of a method in accordance with claim 14 when run on a processor which is operatively coupled with:

a pressure actuator for applying a varying pressure to a body part of a user, and an ultrasound sensing unit for acquiring ultrasound data of an artery in the body part during application of the pressure to the body part.

Subject 1
75 mmHg

Subject 2
75 mmHg

Subject 3
75 mmHg

Subject 4
75 mmHg

FIG. 1

100 mmHg          125 mmHg

(a)               (b)

82                82

(c)               (d)

82                82

FIG. 2

FIG. 3

FIG. 4

10〜

| Control pressure cycle | 〜 12 |

↓

| Acquire U/S data | 〜 14 |

↓

| Segment artery cross-section | 〜 16 |

↓

| Determine artery cross-section area signal | 〜 18 |

↓

| Construct artery area signal | 〜 20 |

↓

| Retrieve arterial collapse model | 〜 22 |

↓

| Fit arterial collapse model to artery area signal | 〜 24 |

↓

| Output measure of arterial pressure | 〜 26 |

FIG. 5

FIG. 6

FIG. 7

FIG. 8

FIG. 9

FIG. 10

Europäisches
Patentamt

European
Patent Office

Office européen
des brevets

# EUROPEAN SEARCH REPORT

Application Number

**EP 23 20 8577**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/360412 A1 (AGUREN DERRICK [US]) 20 December 2018 (2018-12-20) * paragraphs [0023] - [0047]; claims; figures * | 1-15 | INV. A61B8/02 A61B8/00 A61B8/08 |
| A | US 2021/093287 A1 (MASHOOD NABEEL PILAPARAMBIL [IN] ET AL) 1 April 2021 (2021-04-01) * paragraphs [0009] - [0013], [0039], [0042], [0054] * | 1-15 | |
| A | US 2010/106016 A1 (ORBAY JORGE L [US] ET AL) 29 April 2010 (2010-04-29) * paragraphs [0006] - [0020]; figures * | 1-15 | |
| A | US 2017/042431 A1 (MALTZ JONATHAN S [US]) 16 February 2017 (2017-02-16) * paragraph [0013]; figures * | 1-15 | |
| A | US 2019/069842 A1 (ROTHBERG JONATHAN M [US] ET AL) 7 March 2019 (2019-03-07) * paragraphs [0018] - [0020] * | 1-15 | TECHNICAL FIELDS SEARCHED (IPC) A61B |

The present search report has been drawn up for all claims

| Place of search | Date of completion of the search | Examiner |
|---|---|---|
| Munich | 16 April 2024 | Mundakapadam, S |

EPO FORM 1503 03.82 (P04C01)

## ANNEX TO THE EUROPEAN SEARCH REPORT
## ON EUROPEAN PATENT APPLICATION NO.

EP 23 20 8577

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2024

| Patent document cited in search report | | | Publication date | Patent family member(s) | | | Publication date |
|---|---|---|---|---|---|---|---|
| US 2018360412 | A1 | | 20-12-2018 | US | 2016095572 | A1 | 07-04-2016 |
| | | | | US | 2018360412 | A1 | 20-12-2018 |
| | | | | WO | 2016057233 | A1 | 14-04-2016 |
| US 2021093287 | A1 | | 01-04-2021 | AU | 2019283732 | A1 | 17-09-2020 |
| | | | | AU | 2019283733 | A1 | 17-09-2020 |
| | | | | AU | 2019283734 | A1 | 17-09-2020 |
| | | | | AU | 2019283735 | A1 | 17-09-2020 |
| | | | | EP | 3716856 | A1 | 07-10-2020 |
| | | | | EP | 3716857 | A1 | 07-10-2020 |
| | | | | EP | 3716858 | A1 | 07-10-2020 |
| | | | | EP | 3716859 | A1 | 07-10-2020 |
| | | | | US | 2021077057 | A1 | 18-03-2021 |
| | | | | US | 2021077058 | A1 | 18-03-2021 |
| | | | | US | 2021093287 | A1 | 01-04-2021 |
| | | | | WO | 2019234764 | A1 | 12-12-2019 |
| | | | | WO | 2019234765 | A1 | 12-12-2019 |
| | | | | WO | 2019234766 | A1 | 12-12-2019 |
| | | | | WO | 2019234767 | A1 | 12-12-2019 |
| US 2010106016 | A1 | | 29-04-2010 | US | 2010106016 | A1 | 29-04-2010 |
| | | | | WO | 2010048528 | A2 | 29-04-2010 |
| US 2017042431 | A1 | | 16-02-2017 | AU | 2016308466 | A1 | 08-03-2018 |
| | | | | CA | 2995472 | A1 | 23-02-2017 |
| | | | | CN | 108135506 | A | 08-06-2018 |
| | | | | EP | 3334336 | A1 | 20-06-2018 |
| | | | | JP | 2018525106 | A | 06-09-2018 |
| | | | | US | 2017042431 | A1 | 16-02-2017 |
| | | | | US | 2017367592 | A1 | 28-12-2017 |
| | | | | US | 2018325389 | A1 | 15-11-2018 |
| | | | | WO | 2017031005 | A1 | 23-02-2017 |
| US 2019069842 | A1 | | 07-03-2019 | AU | 2018330436 | A1 | 06-02-2020 |
| | | | | CA | 3070482 | A1 | 14-03-2019 |
| | | | | EP | 3678555 | A1 | 15-07-2020 |
| | | | | TW | 201919542 | A | 01-06-2019 |
| | | | | US | 2019069842 | A1 | 07-03-2019 |
| | | | | WO | 2019051007 | A1 | 14-03-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 4173556 A1

**Non-patent literature cited in the description**

- **DRZEWIECKI, G** ; **HOOD, R** ; **APPLE, H**. Theory of the oscillometric maximum and the systolic and diastolic detection ratios. *Ann Biomed Eng*, 1994, vol. 22, 88-96
- *Hindawi BioMed Research International*, vol. 2018
- **DRZEWIECKI, G** ; **HOOD, R.** ; **APPLE, H.** Theory of the oscillometric maximum and the systolic and diastolic detection ratios. *Ann Biomed Eng*, 1994, vol. 22, 88-96
- **RODRÍGUEZ-NIEDENFÜHR M** ; **VÁZQUEZ T** ; **NEAM L** ; **FERREIRA B** ; **PARKIN I** ; **SAÑUDO JR**. Variations of the arterial pattern in the upper limb revisited: a morphological and statistical study, with a review of the literature. *J Anat.*, November 2001, vol. 199, 547-66
- **ALEXANDER H** ; **COHEN ML** ; **STEINFELD L.** Criteria in the choice of an occluding cuff for the indirect measurement blood pressure. *Med Biol Eng Comput.*, January 1977, vol. 15 (1), 2-10
- **ALEXANDER H** ; **COHEN ML** ; **STEINFELD L**. Criteria in the choice of an occluding cuff for the indirect measurement blood pressure. *Med Biol Eng Comput*, January 1977, vol. 15 (1), 2-10